# EUROPÄISCHE PATENTSCHRIFT

(11) **EP 1 572 039 B1**
(45) Veröffentlichungstag und Bekanntmachung des Hinweises auf die Patenterteilung: **07.05.2008**
(21) Anmeldenummer: 02782619.7
(22) Anmeldetag: 17.12.2002
(51) Int. Cl.: A61F 2/44

(54) **ZWISCHENWIRBELIMPLANTAT**
INTERVERTEBRAL IMPLANT
IMPLANT INTERVERT BRAL

(43) Veröffentlichungstag der Anmeldung: 14.09.2005
(73) Patentinhaber: Synthes GmbH, 4436 Oberdorf (CH)
(72) Erfinder: FRIGG, Robert, CH-2544 Bettlach (CH); LECHMANN, Beat, CH-2544 Bettlach (CH)
(74) Vertreter: Lusuardi, Werther
(86) Internationale Anmeldenummer: PCT/CH2002/000708
(87) Internationale Veröffentlichungsnummer: WO 2004/054479

(56) Entgegenhaltungen:
- EP-A- 0 282 161
- WO-A-00/74606
- WO-A-99/59492
- US-A- 5 514 183
- US-A- 5 725 597
- US-A1- 2002 052 656

## Beschreibung

Die Erfindung bezieht sich auf ein Zwischenwirbelimplantat gemäss dem Oberbegriff des Patentanspruchs 1 und auf ein Verfahren zum Ersetzen einer defekten, natürlichen Bandscheibe durch ein Zwischenwirbelimplantat gemäss dem Patentanspruch 23.

Nach Entfernung einer beschädigten, natürlichen Bandscheibe oder eines beschädigten Nukleus pulposus einer Bandscheibe werden Implantate oder Prothesen in den Zwischenwirbelraum zweier benachbarter Wirbelkörper eingebracht. Dabei entsteht das Ziel, wieder möglichst natürliche Zustände herbeizuführen, d.h. insbesondere die ursprüngliche Bandscheibenhöhe und damit den ursprünglichen Abstand zwischen den beiden benachbarten Wirbelkörpern wiederherzustellen. Ferner sollen Bewegungen der benachbarten Wirbelkörper relativ zueinander möglichst ohne Behinderung in ihrer natürlichen Art ausführbar sein. Hierzu ist die Erhaltung der Bewegungsmöglichkeiten bei einer Vorwärts/Rückwärtsneigung, d.h. Flexion und Extension der Wirbelkörper sowie bei einer lateralen Beugung der Wirbelkörper innerhalb der natürlichen Grenzen wesentlich. Die natürlichen Bänder und Muskeln entlang der Wirbelsäule werden im wesentlichen intakt gelassen, so dass diese die Bewegungen eines mechanischen Bandscheibenersatzes weiter stabilisieren.

Eine gattungsgemässe Bandscheibenendoprothese gemäß dem Oberbegriff des Anspruch 1 ist aus der DE-A 35 29 761 BÜTTNER bekannt. Diese bekannte Bandscheibenendoprothese besteht im wesentlichen aus zwei symmetrischen Abschlussplatten mit gegeneinander gerichteten konkaven Gleitflächen und je einer aussenstehenden Oberfläche zur Anlage an die Grundplatte, respektive die Deckplatte der angrenzenden Wirbelkörper und einem zwischen den Abschlussplatten positionierten Distanzstück mit zu den konkaven Gleitflächen an den Abschlussplatten komplementär ausgestalteten konvexen Gleitflächen. Die Gleitflächen sind in einer Ausführungsform als Teilflächen einer Zylindermantelfläche ausgebildet, wobei die an den beiden Abschlussplatten angeordneten Gleitflächen komplementär zu je einer der angrenzenden Gleitflächen am Distanzstück ausgestaltet sind und je zwei komplementäre Gleitflächen die aufeinander verschiebbaren Artikulationsflächen eines um eine Drehachse rotierbaren Gelenkteiles bilden. Das Gelenk umfasst ein oberes und ein unteres Gelenkteil mit je einer Drehachse. Die beiden Drehachsen sind um 90° zueinander versetzt. Nachteilig an dieser bekannten Bandscheibenendoprothese ist, dass
a) den durch die natürliche Bandscheibe übertragbaren überlagerten Schwenkbewegungen insbesondere bei anterior-posteriorer und bei lateraler Flexion, welche bei der natürlichen Bandscheibe unabhängig voneinander sind, durch die Ausgestaltung einer Bandscheibenendoprothese mit nur einem Drehzentrum nicht Rechnung getragen wird;
b) nachteilige Reibungskräfte bei zwei aufeinander gleitbaren, artikulierenden Flächen entstehen. Ferner sind an den Flächen Verschleiss, d.h. unter anderem auch Abrieb sowie Widerstand bei der Bewegung der Gelenkteile die Folge. Zudem besteht das Risiko des "Stick-Slip" Effektes; und
c) ein mechanischer Bandscheibenersatz die weitere Degeneration der betroffenen Bewegungssegmente kaum aufhalten kann. Das Wiederherstellen der ursprünglichen Bewegungsverhältnisse reduziert den Schmerz wesentlich und der Patient gewinnt an Lebensqualität. Bei erneutem Auftreten von Schmerz muss jedoch eine Revision der Versorgung in Angriff genommen werden. Dabei wird üblicherweise eine Bandscheibenprothese nach herkömmlicher Bauart komplett entfernt und das Bewegungssegment versteift. Diese Operation belastet den Patienten ausserordentlich.

Hier will die Erfindung Abhilfe schaffen. Der Erfindung liegt die Aufgabe zugrunde, ein Zwischenwirbelimplantat zu schaffen, welches ein Gelenk umfasst, dessen Gelenkachsen Lagerungen mit einer minimalen Reibung aufweisen.

Die Erfindung löst die gestellte Aufgabe mit einem Zwischenwirbelimplantat, welches die Merkmale des Anspruchs 1 aufweist.

Die durch die Erfindung erreichten Vorteile sind im wesentlichen darin zu sehen, dass dank des erfindungsgemässen Zwischenwirbelimplantates
- Die Schwenkbewegungen in antero-posteriorer Richtung und nach lateral unabhängig sind;
- die Reibfläche der Bewegungen auf den insgesamt vier linienförmigen Kontakten auf ein Minimum reduziert ist; und
- durch die infolge des Linienkontaktes zwischen den Gelenkteilen anstelle von Gleitflächen geringere Reibungskräfte im Gelenk auftreten und daher Relativbewegungen der Wirbelkörper, insbesondere die laterale Beugung und Flexions- / Extensionsbewegungen der Wirbelsäule nicht behindert werden.

In einer weiteren Ausführungsform des erfindungsgemässen Zwischenwirbelimplantates sind zwei einander gegenüberliegende Oberflächen oder beide Paare einander gegenüberliegender Oberflächen als Gleitflächen für den oder die kreiszylindrischen Stäbe ausgebildet. Dabei können diese Gleitflächen als planare, kreiszylindrische oder kegelförmige Flächen ausgebildet sein.

Die Vorteile der verschiedenen Ausgestaltungen der Gleitflächen liegen darin, dass
- planare Gleitflächen den kreiszylindrischen Stäben eine uneingeschränkte Bewegung bei einer Neigung der angrenzenden Wirbelkörper relativ zueinander sowie bezüglich einer Translationsbewegung der angrenzenden Wirbelkörper relativ zueinander gestatten;
- konkave oder insbesondere kreiszylindrische erlauben, dass je nach Bewegungssegment der Wirbelsäule den physiologischen Neigebewegungen der angrenzenden Wirbelkörper Rechnung getragen wird; und
- geneigte Gleitflächen gestatten gleichzeitig mit der Operation eine Korrektur der Lordose oder Kyphose.

In einer anderen Ausführungsform des erfindungsgemässen Zwischenwirbelimplantates sind die als Gleitflächen ausgestalteten Oberflächen an den drei plattenförmigen Teilen mit einer peripheren Umrandung als Sicherung für die Stäbe versehen. Damit ist der

Vorteil erreichbar, dass durch die Umrandung die kreiszylindrischen Stäbe gegen ein Herausfallen oder ein Herausgequetschwerden aus den Zwischenräumen zwischen den drei plattenförmigen Teilen gesichert sind.

In wiederum einer weiteren Ausführungsform des erfindungsgemässen Zwischenwirbelimplantates sind mindestens auf einem Teil der Gleitflächen eine Anzahl Begrenzer/Stops für die Einschränkung der Rotation der zylindrischen Stäbe um die Zentralachse vorgesehen. Durch diese Ausgestaltung sind folgende Vorteile erreichbar:
- Begrenzung der Rotation der beiden Stäbe auf eine bestimmte Richtung aber mit einem angulären Spielraum;
- Einstellbarkeit dieser Richtung auf anterio-posterior für den einen Stab und medio-lateral für den anderen Stab; und
- Verhinderung, dass sich die beiden Stäbe parallel zueinander ausrichten, so dass das Gelenk des Implantates zwei voneinander beabstandete, parallele Drehachsen aufweisen würde und dadurch die beiden an das Zwischenwirbelimplantat angrenzenden Wirbelkörper nur Flexions/Extensionsbewegungen und keine lateralen Beugungen oder umgekehrt ausführen könnten.

In einer anderen Ausführungsform sind auf einem oder beiden der durch die vier Gleitflächen gebildeten Gleitflächen-Paare ein Paar Nuten als Lager für den ersten und/oder zweiten Stab vorgesehen sind. Vorzugsweise ist jedes Paar Nuten kongruent zu den darin aufzunehmenden, kreiszylindrischen Stäben. Der Vorteil dieser Ausführungsform liegt darin, dass durch die Positionierung der Nuten die Neigung der angrenzenden Wirbelkörper nur streng in den vorgegebenen Richtungen wie laterales Neigen, sowie Flexion und Extension einstellbar ist. Scherkräfte, welche auf die Wirbelgelenke wirken können, lassen sich durch das Zwischenwirbelimplantat aufgefangen, da keine Translationsbewegungen der an die Wirbelkörper angrenzenden plattenförmigen Teile möglich ist.

In wiederum einer anderen Ausführungsform ist mindestens ein Paar Nuten inkongruent zu den darin aufzunehmenden, kreiszylindrischen Stäben ausgestaltet und weist vorzugsweise eine Breite auf, welche eine begrenzte Rotation der Stäbe um die Zentralachse in den Nuten zulässt. Der Vorteil dieser Ausführungsform liegt in der Begrenzung der Bewegungsmöglichkeiten der angrenzenden Wirbelkörper auf Neigungen. Gleichzeitig kann eine Translation streng lateral oder streng antero-posterior erlaubt werden.

In einer weiteren Ausführungsform weist mindesten ein Teil der Nuten eine peripher angebrachte Begrenzung/Stop gegen eine axiale Verschiebung des darin aufgenommenen Stabes auf. Vorzugsweise münden die Nuten nicht in die Seitenflächen der plattenförmigen Teile sondern sind an ihren Enden geschlossen. Dadurch ist erreichbar, dass die kreiszylindrischen Stäbe nicht parallel zu ihren Längsachsen aus den Nuten herausrutschen können.

Vorzugsweise verläuft das eine Paar Nuten für den ersten Stab von den ventralen zu den dorsalen Seitenflächen der dazugehörenden plattenförmigen Teile während das zweite Paar Nuten für den zweiten Stab zwischen den lateralen Seitenflächen der dazugehörenden plattenförmigen Teile verläuft.

Durch die antero-posteriore Ausrichtung der Längsachse des ersten Stabes und laterale Ausrichtung der Längsachse des zweiten Stabes ergibt sich ein Gelenk mit gekreuzten Drehachsen. Die Nuten sind vorzugsweise derart angeordnet, dass einmal der Stab mit der antero-posterior gerichteten Längsachse oben ist und der Stab mit der lateral-lateral gerichteten Längsachse unten ist. Die Umkehrung ist aber auch möglich, so dass dem Umstand Rechnung getragen werden kann, dass die einzelnen Bewegungssegmente der Wirbelsäule naturgemäss verschiedene Achslagen zeigen.

Statt durch Nuten kann diese Ausrichtung der Stäbe auch durch die Anordnung der Begrenzer/Stops erfolgen.

In einer anderen Ausführungsform umfasst dieses elastisch deformierbare Mittel, welche das obere und untere Teil mit dem dazwischen liegenden mittleren Teil und den beiden Stäben untereinander zusammenhalten. Die elastisch deformierbaren Mittel können Federn oder elastomere Verbindungselemente sein.

In einer weiteren Ausführungsform sind die vier Gleitflächen und die beiden Stäbe metallisch.

In wiederum einer weiteren Ausführungsform des erfindungsgemässen Zwischenwirbelimplantates sind die vier Gleitflächen metallisch und die beiden Stäbe keramisch.

Für die plattenförmigen Teile und die zylindrischen Stäbe sind die folgenden Abmessungen geeignet:
- Länge der kreiszylindrischen Stäbe: grösser als die Hälfte der Ausdehnung der mit dem Stab in Berührung stehenden Gleitfläche;
- Radius der kreiszylindrischen Stäbe: zwischen 0,3 mm und 5,0 mm;
- Zylinderradius der Gleitflächen: zwischen 12 mm und 140 mm;
- Breite der Nuten: zwischen 3 mm und 12 mm;
- Tiefe der Nuten: zwischen 0,2 mm und 4,8 mm; und
- Winkelbereich der zugelassenen Rotation der kreiszylindrischen Stäbe um die Zentralachse des Zwischenwirbelimplantates: zwischen 1° und 32°.

In einer anderen Ausführungsform sind von den ventralen Seitenflächen her Mittel an den drei plattenförmigen Teilen anbringbar, wodurch die drei plattenförmigen Teile ventral auf einer bestimmten Distanz relativ zueinander gehalten werden können. Dadurch ist der Vorteil erreichbar, dass die drei plattenförmigen Teile zur Einführung in den Zwischenwirbelraum in eine Position mit fest gehaltener Implantathöhe bringbar sind und nach der Einführung in den Zwischenwirbelraum um das Gelenk bewegbar und an die Grund- respektive Deckplatte der angrenzenden Wirbelkörper zur Anlage bringbar sind.

In einer weiteren Ausführungsform ermöglichen die Mittel eine temporäre Blockierung der Beweglichkeit der drei plattenförmigen Teile um die Gelenke. Dadurch ist der Vorteil erreichbar, dass mittels eines minimal invasiven Eingriffes die im Zwischenwirbelraum integrierten Gelenke blockierbar sind. Dies ist besonders vorteilhaft in Fällen bei denen post-operativ Schmerzen auftreten, d.h. wo die Degeneration des betroffenen Wirbelsäulensegmentes weitergeht und der Chirurg eine Fusion der betroffenen Wirbel in Betracht zieht. Vorzugsweise sind die Mittel an den ventralen Seitenflächen der drei plattenförmigen Teile anbringbar. Durch dieses spätere, sekundäre Blockieren der Bewegbarkeit der drei plattenförmigen Teile um die Gelenke wird das Zwischenwirbelimplantat versteift und in ein Arthrodesenimplantat (Fusions-Käfig) übergeführt.

In wiederum einer weiteren Ausführungsform umfassen die Mittel einen Einsatz, welche in je eine Vertiefung an den einander gegenüberliegenden Oberflächen des oberen und unteren plattenförmigen Teiles einsetzbar ist. Vorzugsweise sind die Vertiefungen als Schwalbenschwanzführungen ausgestaltet, welche an den ventralen Seitenflächen der beiden aussenstehenden plattenförmigen Teile offen sind, so dass die zu den Schwalbenschwanzführungen komplementär ausgestalteten Enden des Einsatzes von ventral in die Schwalbenschwanzführungen eingeschoben werden können. Dadurch ist der Vorteil erzielbar, dass durch das Einführen des Einsatzes die Bewegbarkeit der beiden plattenförmigen Teile um das Gelenk blockierbar ist. Die Starrheit der Blockierung lässt sich erhöhen, wenn die Schwalbenschwanzführungen so ausgestaltet sind, dass sie sich gegen die Zentralachse des Zwischenwirbelimplantates verjüngen, so dass der Einsatz zusätzlich in den Schwalbenschwanzführungen verkeilbar ist.

In wiederum einer anderen Ausführungsform sind die beiden plattenförmigen Teile mit Bohrungen zur Aufnahme von Knochenfixationsmittel, insbesondere von Knochenschrauben versehen, wobei die Bohrungen Längsachsen aufweisen, welche schräg zur Zentralachse stehen. Vorzugsweise durchdringen je zwei Bohrungen eines der beiden plattenförmigen Teile von der ventralen Seitenfläche zur Appositionsfläche. Dabei können die Längsachsen, falls nur eine axiale Fixierung des Zwischenwirbelimplantates vorgesehen ist, nur von lateral betrachtet schräg zur Zentralachse stehen, oder falls eine winkelstabile Fixierung des Zwischenwirbelimplantates vorgesehen ist, auch von ventral betrachtet von den inneren Oberflächen der beiden plattenförmigen Teile gegen die Appositionsflächen divergieren.

In einer weiteren Ausführungsform sind die Bohrungen zur Aufnahme der Knochenfixationsmittel mit Innengewinden versehen, wodurch sich eine zusätzliche, rigide Fixierung der Knochenfixationsmittel in den beiden plattenförmigen Teilen erreichen lässt. Vorzugsweise sind die Bohrungen konisch ausgestaltet, so dass durch die konischen Gewindeverbindungen zwischen den Innengewinden und den Aussengewinden an den Köpfen der Knochenfixationsmittel eine verstärkte Fixierung der Knochenfixationsmittel an jedem der beiden plattenförmigen Teile erreichbar ist.

Die Appositionsflächen sind vorzugsweise konvex ausgestaltet und mit einer dreidimensionalen Strukturierung, vorzugsweise in Form von pyramidenförmigen Erhebungen versehen. Durch diese Ausgestaltung der Appositionsflächen wird der Anatomie der Wirbelkörperendplatten Rechnung getragen.

Der Ersatz einer defekten, natürlichen Bandscheibe durch ein Zwischenwirbelimplantat umfasst die Schritte:
A) blockieren des oder der Gelenke eines Zwischenwirbelimplantates mittels dafür vorgesehener Mittel in einer bestimmten Position des Gelenkes;
B) einführen des Zwischenwirbelimplantates in den zu behandelnden Zwischenwirbelraum;
C) lösen und entfernen der zur Blockierung des Gelenkes in das Zwischenwirbelimplantat eingesetzten Mittel. Durch die Blockierung des Gelenkes ist der Vorteil erreichbar, dass die beweglichen plattenförmigen Teile mit den aussenstehenden Appositionsflächen einfacher in den zu behandelnden Zwischenwirbelraum einführbar sind.

In einer weiteren Anwendung des Verfahren umfasst dieses das nachträgliche Blockieren des Gelenkes am implantierten Zwischenwirbelimplantat mittels der zur Blockierung des Gelenkes vorgesehenen Mittel. Dadurch ist der Vorteil erreichbar, dass bei einem Auftreten von post-operativen Schmerzen für den Patienten oder bei einer weiteren Degeneration des betroffenen Bewegungssegmentes das Gelenk am Zwischenwirbelimplantat postoperativ durch Einsetzen der dazu vorgesehenen Mittel blockierbar sind. Diese nachträgliche Blockierung ist mit einem minimal-invasiven, vorzugsweise einem laparoskopischen Eingriff möglich. Das Zwischenwirbelimplantat übernimmt dann die Aufgabe eines Käfigs, so dass das betroffene Bewegungssegment der Wirbelsäule versteift werden kann.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine Explosionsdarstellung einer Ausführungsform des erfindungsgemässen Zwischenwirbelimplantates;
Fig. 2a - 2c drei perspektivische Darstellungen verschiedener Ausführungsformen der Gleitflächen am Beispiel des unteren plattenförmigen Teiles;
Fig. 3a eine Aufsicht auf eine Ausführungsform des erfindungsgemässen Zwischenwirbelimplantates;
Fig. 3b einen Schnitt parallel zur zweiten Drehachse der in Fig. 3a dargestellten Ausführungsform des erfindungsgemässen Zwischenwirbelimplantates;
Fig. 4 eine Explosionsdarstellung einer weiteren Ausführungsform des erfindungsgemässen Zwischenwirbelimplantates;
Fig. 5 einen Schnitt parallel zur zweiten Drehachse der in Fig. 4 dargestellten Ausführungsform des erfindungsgemässen Zwischenwirbelimplantates;
Fig. 6 eine Explosionsdarstellung einer weiteren Ausführungsform des erfindungsgemässen Zwischenwirbelimplantates;
Fig. 7 eine Explosionsdarstellung einer weiteren Ausführungsform des erfindungsgemässen Zwischenwirbelimplantates;
Fig. 8a eine Aufsicht auf die in Fig. 7 dargestellte Ausführungsform des erfindungsgemässen Zwischenwirbelimplantates;
Fig. 8b einen Schnitt parallel zur zweiten Drehachse der in den Fig. 7 und 8a dargestellten Ausführungsform des erfindungsgemässen Zwischenwirbelimplantates;
Fig. 9 eine Explosionsdarstellung einer weiteren Ausführungsform des erfindungsgemässen Zwischenwirbelimplantates;
Fig. 10a eine Aufsicht auf die in Fig. 9 dargestellte Ausführungsform des erfindungsgemässen Zwischenwirbelimplantates;
Fig. 10b einen Schnitt parallel zur zweiten Drehachse der in den Fig. 9 und 10a dargestellten Ausführungsform des erfindungsgemässen Zwischenwirbelimplantates;
Fig. 11 eine perspektivische Ansicht einer weiteren Ausführungsform des erfindungsgemässen Zwischenwirbelimplantes; und
Fig. 12 eine perspektivische Ansicht einer Ausführungsform des erfindungsgemässen Zwischenwirbelimplantes im implantierten Zustand.

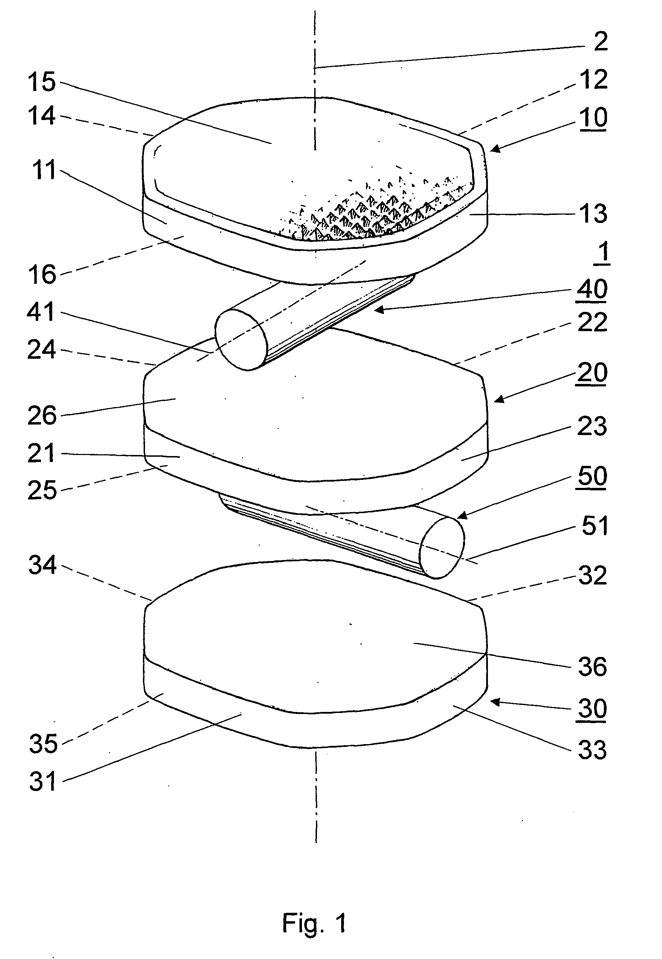

In Fig. 1 ist eine Ausführungsform des erfindungsgemässen Zwischenwirbelimplantates 1 dargestellt, welche bezüglich der Zentralachse 2 übereinander angeordnet ein oberes Teil 10, mit einer ventralen, einer dorsalen und zwei lateralen Seitenflächen 11;12;13;14 sowie quer zur Zentralachse 2 angeordnet einer oberen Appositionsfläche 15 und einer unteren Oberfläche 16, ein unteres Teil 30, mit einer ventralen, einer dorsalen und zwei lateralen Seitenflächen 31;32;33;34 sowie quer zur Zentralachse 2 angeordnet einer unteren Appositionsfläche 35 und einer oberen Oberfläche 36, und zwischen dem oberen und dem unteren Teil 10;30 ein mittleres Teil 20, mit einer ventralen, einer dorsalen und zwei lateralen Seitenflächen 21;22;23;24 sowie gegenüber dem unteren Teil 30 mit einer unteren Oberfläche 25 und gegenüber dem oberen Teil 10 einer oberen Oberfläche 26 umfasst. Die drei plattenförmigen Teile 10;20;30 weisen orthogonal zur Zentralachse 2 eine ovale, elliptische, kreisförmige oder polygonale Querschnittsfläche auf. Die paarweise einander zugewandten Oberflächen 16;26;25;35 der drei plattenförmigen Teile 10;20;30 sind hier als ebene Flächen ausgebildet. Zwischen der oberen Oberfläche 26 des mittleren Teils 20 und der unteren Oberfläche 16 des oberen Teils 10 ist ein erster kreiszylindrischer Stab 40 eingefügt. Ferner ist zwischen der unteren Oberfläche 25 des mittleren Teils 2 und der oberen Oberfläche 36 des unteren Teils 30 ein zweiter kreiszylindrischer Stab 50 eingefügt. Die beiden kreiszylindrischen Stäbe 40;50 sind derart zwischen den Oberflächen 16;26;25;36 angeordnet, dass ihre Längsachsen 41;51 senkrecht zur Zentralachse 2 stehen. Durch die zwischen den Oberflächen 16;26;25;36 angeordneten kreiszylindrischen Stäbe 40;50 wird eine Rotierbarkeit des oberen Teils 10 relativ zum unteren Teil 30 um die Längsachsen 41;51 der kreiszylindrischen Stäbe 40;50 hergestellt.

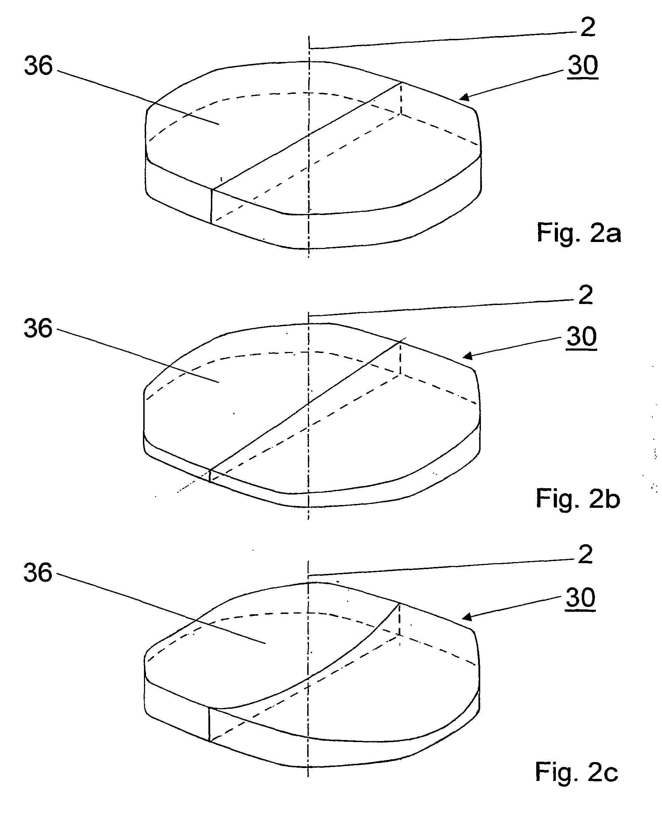

In den Fig. 2a bis 2c sind verschiedene Ausführungsformen der als Gleitfläche dienenden Oberfläche 36 am Beispiel des unteren plattenförmigen Teiles 30 dargestellt. Dabei ist die Oberfläche 36 in Fig. 2a senkrecht zur Zentralachse 2 planar und in Fig. 2c konkav und kreiszylindrisch ausgestaltet. Das untere plattenförmige Teil 30 gemäss Fig. 2b ist keilförmig ausgestaltet, wobei die Oberfläche 36 planar ausgestaltet ist und nicht senkrecht zur Zentralachse 2 steht. Die als Gleitflächen dienenden Oberflächen 16;26;25 des oberen und des mittleren plattenförmigen Teiles 10;20 können analog ausgestaltet sein.

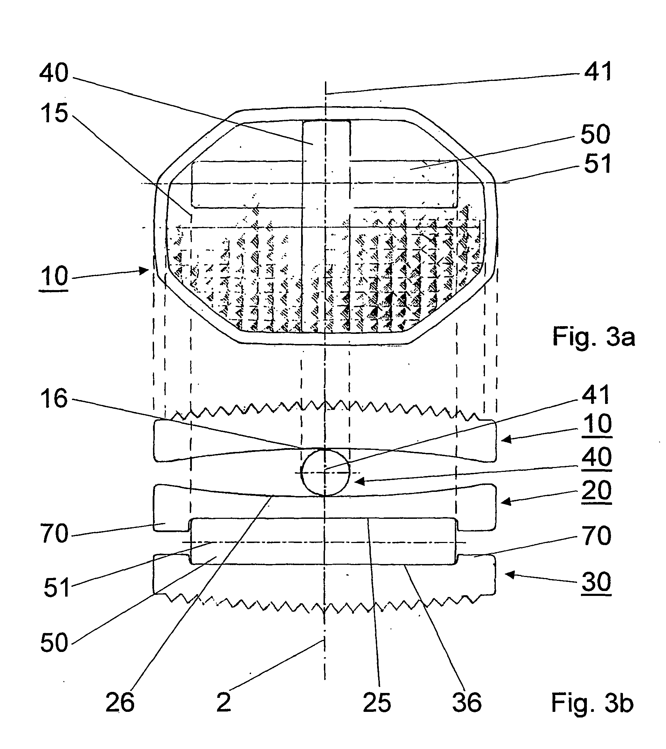

Die in den Fig. 3a und 3b dargestellte Ausführungsform des erfindungsgemässen Zwischenwirbelimplantates 1 unterscheidet sich von der in Fig. 1 dargestellten Ausführungsform nur darin, dass die als Gleitflächen dienenden Oberflächen 16;25;26;36 der drei plattenförmigen Teile 10;20;30 wie in Fig. 2 c dargestellt konkav und kreiszylindrisch ausgestaltet sind. Ferner sind die Oberflächen 16;25;26;36 so gewölbt, dass die Längsachse 41 des ersten kreiszylindrischen Stabes 40 antero-posterior verläuft die Zentralachse 2 des Zwischenwirbelimplantates 1 schneidet, während die Längsachse 51 des zweiten kreiszylindrischen Stabes 50 medio-lateral verläuft und gegenüber der Längsachse 2 des Zwischenwirbelimplantates 1 beabstandet ist. Ferner sind die Oberflächen 16;25;26;36 mit einer teilweisen Umrandung 70 versehen, welche senkrecht zu den Längsachsen 41;51 der zwei kreiszylindrischen Stäben 40;50 angeordnet sind, und eine Verschiebung der Stäbe 40;50 parallel zu ihren Längsachsen 41;51 verhindern.

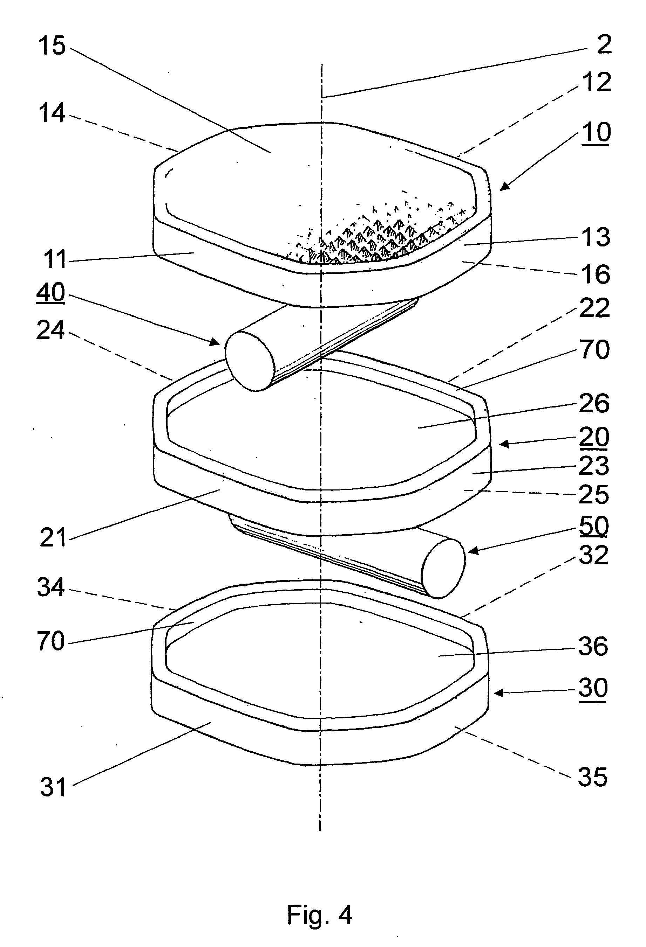

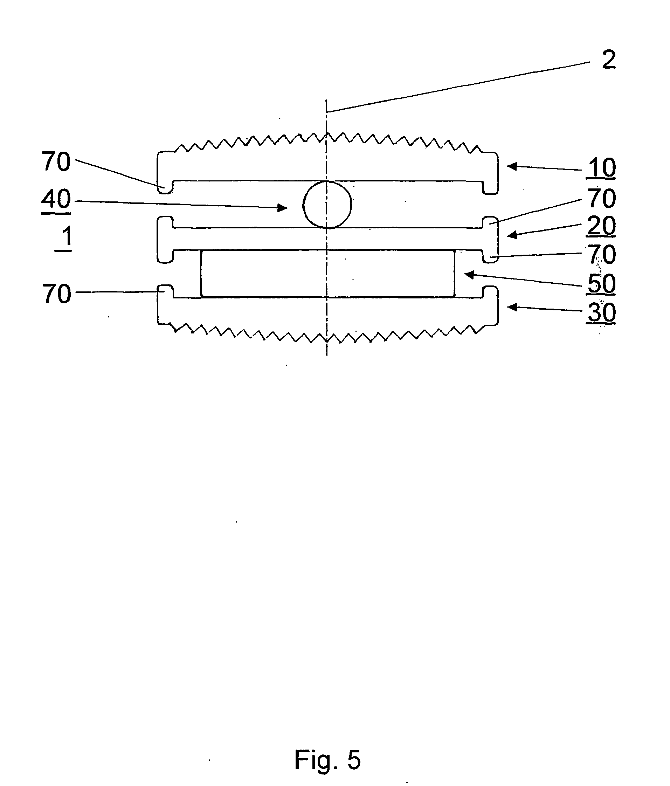

In den Fig. 4 und 5 ist eine Ausführungsform des erfindungsgemässen Zwischenwirbelimplantates dargestellt, welche sich von der in Fig. 1 dargestellten Ausführungsform nur darin unterscheidet, dass die als Gleitflächen ausgestalteten Oberflächen 16;25;26;36 an den drei plattenförmigen Teilen 10;20;30 mit einer peripheren Umrandung 70 versehen sind.

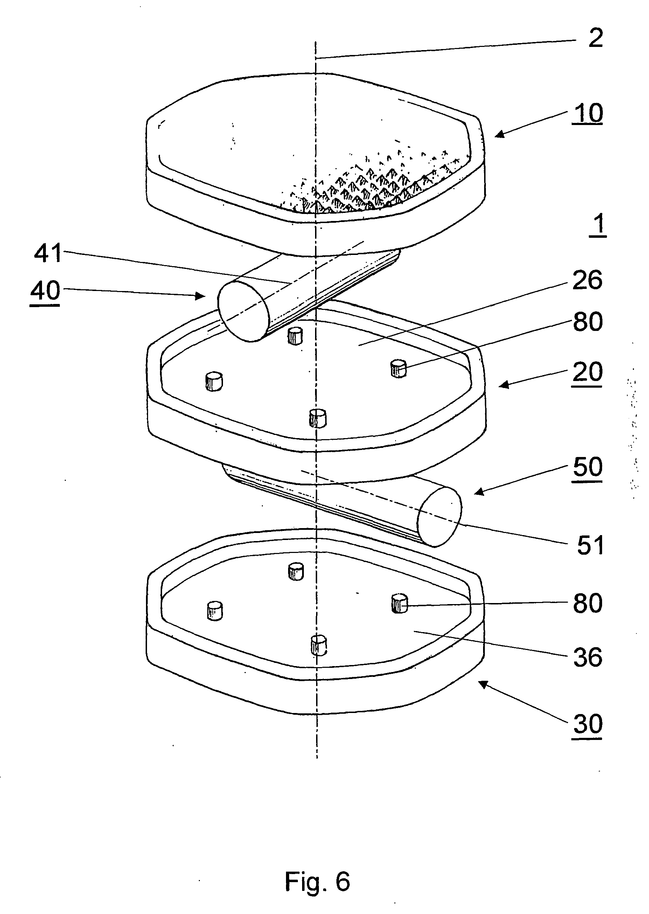

Die in Fig. 6 dargestellte Ausführungsform des erfindungsgemässen Zwischenwirbelimplantates 1 unterscheidet sich von der in den Fig. 4 und 5 dargestellten Ausführungsform nur dadurch, dass auf der oberen Oberfläche 26 des mittleren plattenförmigen Teiles 20 vier Begrenzer/Stops 80 für die Einschränkung der Bewegung des ersten kreiszylindrischen Stabes 40 zwischen dem oberen und dem mittleren Teil 10;20 und auf oberen Oberfläche 36 des unteren plattenförmigen Teiles 30 ebenfalls vier Begrenzer/Stops 80 für die Einschränkung der Bewegung des zweiten kreiszylindrischen Stabes 50 angebracht sind. Durch die Begrenzer/Stops 80 wird der Drehwinkel der Längsachsen 41;51 der beiden Stäbe 40;50 um die Zentralachse 2 eingeschränkt.

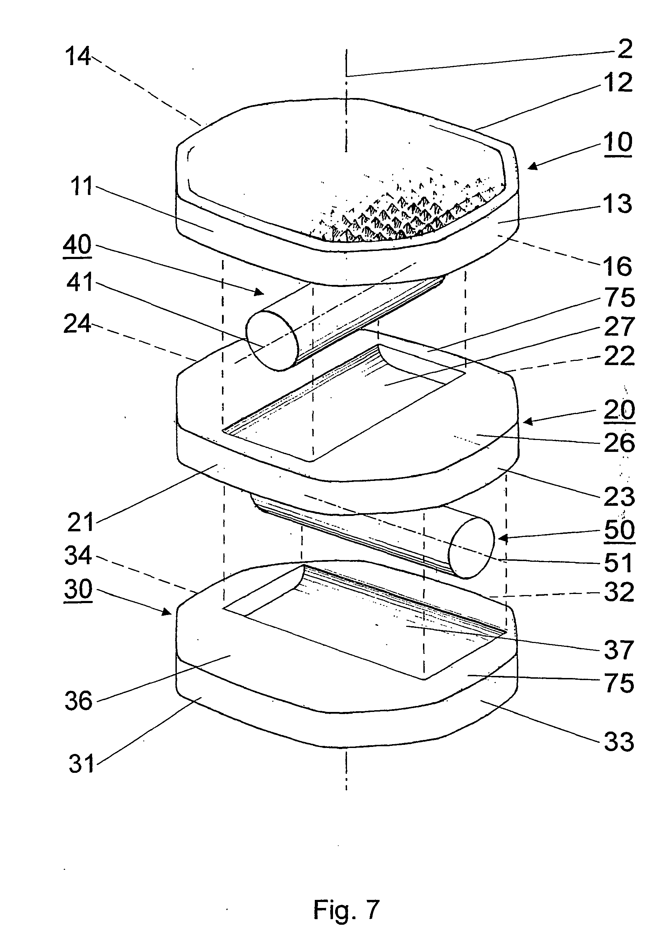

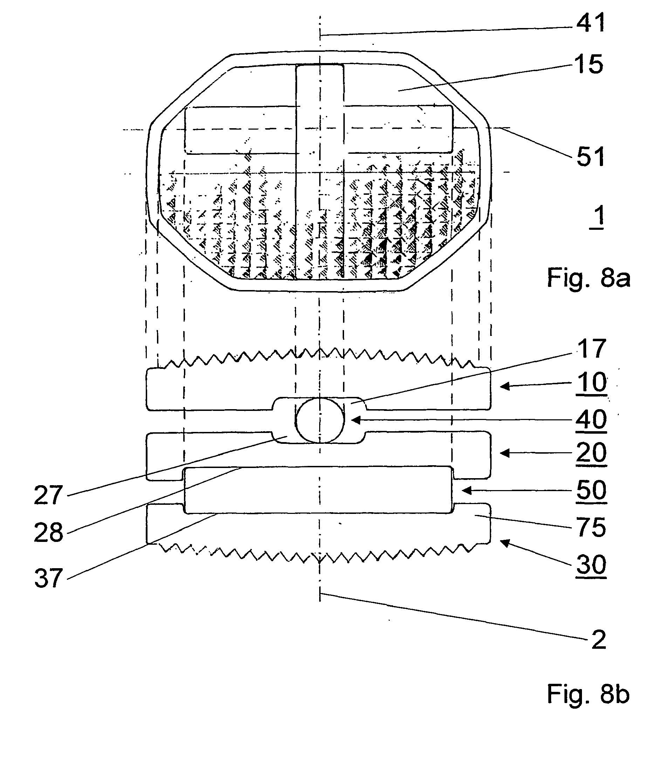

In den Fig. 7 und 8 ist eine Ausführungsform des erfindungsgemässen Zwischenwirbelimplantates 1 dargestellt, bei welcher die paarweise einander zugeordneten Oberflächen 16;26;25;36 der drei plattenförmigen Teile 10;20;30 mit quer zur Zentralachse 2 verlaufenden Nuten 17;27;28;37 versehen sind. Die Nuten 17;27;28;37 dienen zur teilweisen Aufnahme der kreiszylindrischen Stäbe 40;50 weisen orthogonal zu den Längsachsen 41;51 der kreiszylindrischen Stäbe 40;50 Querschnittsflächen auf, welche Teilflächen eines Ovals sind. Dabei sind die Nuten 17;27, welche zur Aufnahme des ersten kreiszylindrischen Stabes 40 dienen, so angeordnet, dass die Längsachse 41 des ersten kreiszylindrischen Stabes 40 in antero-posteriorer Richtung verläuft. Die den zweiten kreiszylindrischen Stab 50 aufnehmenden Nuten 28;37 sind so angeordnet, dass die Längsachse 51 des zweiten kreiszylindrischen Stabes 50 in medio-lateraler Richtung verläuft. Ferner sind die Nuten 17;27;28;37 gegen die Seitenflächen 11;12;21;22;23;24;33;34 der drei plattenförmigen Teile 10;20;30 durch Begrenzungen/Stops 75 geschlossen, so dass die kreiszylindrischen Stäbe 40;50 nicht parallel zu ihren Längsachsen 41;51 aus den Nuten 17;27;28;37 herausgeschoben werden können. Die zwei kreiszylindrischen Stäbe 40;50 werden durch die Nuten 17;27;28;37 teilweise aufgenommen, so dass sie axial geführt werden. Die Nuten 17;27;28;37 sind so angeordnet, dass die antero-posterior verlaufende Längsachse 41 des ersten kreiszylindrischen Stabes 40 diametral angeordnet ist und die Zentralachse 2 schneidet, während die medio-lateral verlaufende Längsachse 51 des zweiten kreiszylindrischen Stabes 50 von der Zentralachse 2 beabstandet ist.

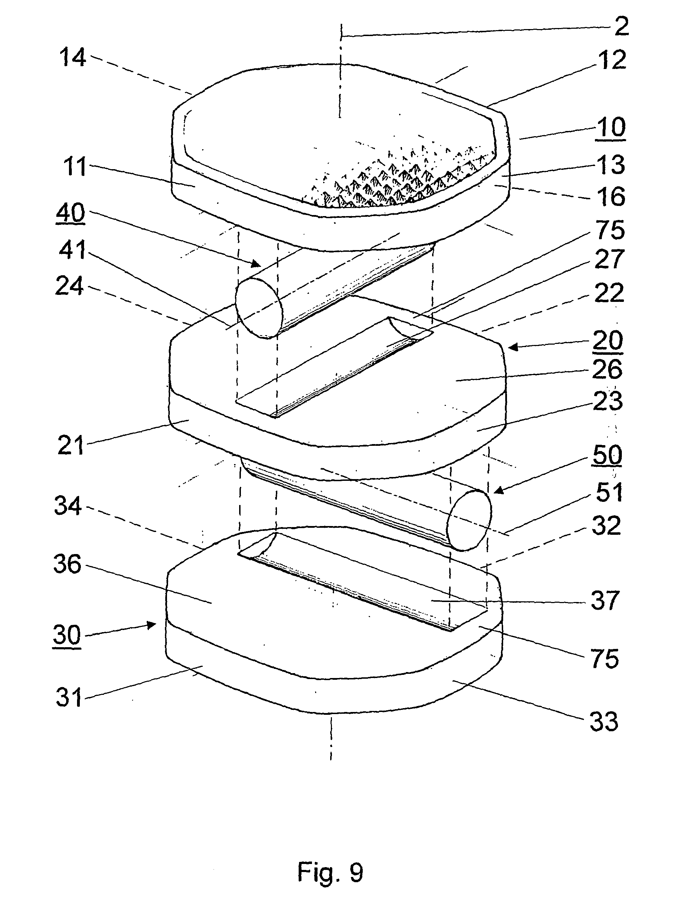

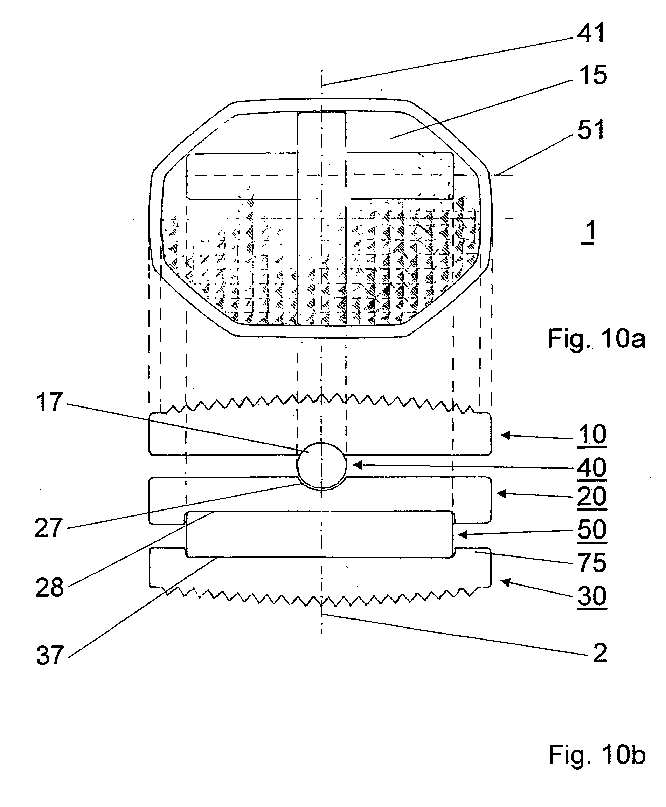

In den Fig. 9 und 10 ist eine Ausführungsform des erfindungsgemässen Zwischenwirbelimplantates 1 dargestellt, welche sich von der in den Fig. 7 und 8 dargestellten Ausführungsform nur darin unterscheidet, dass die Nuten 17;27;28;37 eine zu den Längsachsen 41;51 der kreiszylindrischen Stäbe 40;50 orthogonale Querschnittsfläche aufweisen, welche kreissegmentartig ist. Die zwei kreiszylindrischen Stab 40;50 werden durch die Nuten 17;27;28;37 teilweise aufgenommen, so dass sie sowohl axial als auch quer zu seiner Längsachse 41 durch die beiden Nuten 17;27;28;37 geführt werden und nur eine Rotationsbewegung um ihre Längsachsen 41;51 ausführen können. Die Nuten 17;27;28;37 sind so angeordnet, dass die antero-posterior verlaufende Längsachse 41 des ersten kreiszylindrischen Stabes 40 diametral angeordnet ist und die Zentralachse 2 schneidet, während die medio-lateral verlaufende Längsachse 51 des zweiten kreiszylindrischen Stabes 50 von der Zentralachse 2 beabstandet ist.

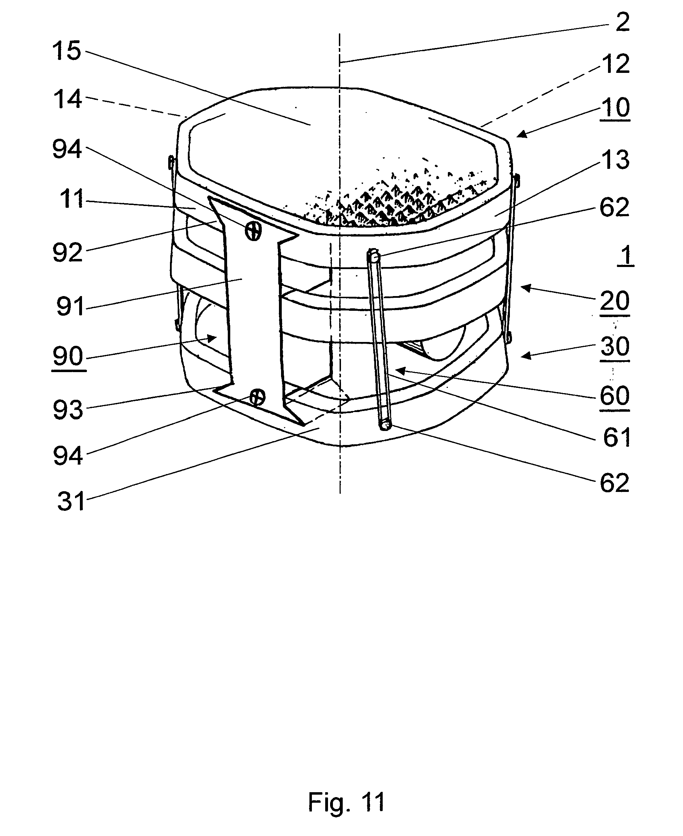

In Fig. 11 dargestellt ist eine Ausführungsform des Zwischenwirbelimplantates, welche sich darin von den in den Fig. 1 bis 10 dargestellten Ausführungsformen unterscheidet, dass das Zwischenwirbelimplantat 1 zusätzlich Mittel 90 umfasst, wodurch die Bewegbarkeit der drei plattenförmigen Teile 10;20;30 relativ zueinander lösbar blockierbar ist. Die Mittel 90 umfassen in der hier dargestellten Ausführungsform einen von ventral her quer zur Zentralachse 2 und parallel zu den lateralen Seitenflächen 13;14;23;24;33;34 der drei plattenförmigen Teile 10;20;30 einschiebbaren Einsatz 91. Das Einschieben des Einsatzes 91 erfolgt in zwei Vertiefungen 92;93, welche als Schwalbenschwanzführungen ausgestaltet sind. Der Einsatz 91 wird von den ventralen Seitenflächen der beiden aussenstehenden plattenförmigen Teile 10;30 in die als Schwalbenschwanzführungen ausgestalteten Vertiefungen 92;93 eingeführt und an beiden plattenförmigen Teilen 10;30 mittels je einer Schraube 94 befestigt. Zudem ist der Einsatz 91 endständig komplementär zu den Vertiefungen 92;93 ausgestaltet, so dass die beiden aussenstehenden plattenförmigen Teile 10;30 bei eingeschobenem Einsatz 91 parallel zur Zentralachse 2 relativ zueinander fixiert sind. Ferner umfasst das Zwischenwirbelimplantat 1 als Federn 61 ausgestaltete, elastisch deformierbare Mittel 60, wodurch die drei plattenförmigen Teile 10;20;30 parallel zur Zentralachse 2 zusammengehalten werden. Wegen der axial elastischen Deformierbarkeit der elastisch deformierbaren Mittel 60 wird die Bewegbarkeit der drei plattenförmigen Teile 10;20;30 um die als Drehachsen dienenden Längsachsen 41;51 der zwei kreiszylindrischen Stäbe 40;50 nicht behindert. Die Federn 61 sind als Zugfedern ausgestaltet und an Nasen 62 befestigt, welche an den Seitenflächen 11;12;13;14; 31;32;33;34 der beiden aussenstehenden plattenförmigen Teile 10;30 angebracht sind.

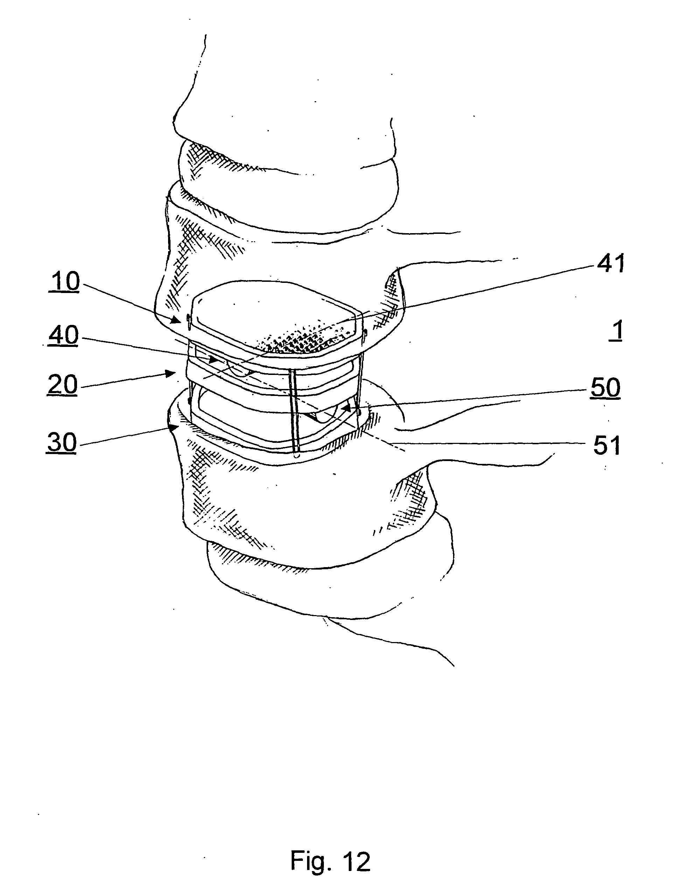

In Fig. 12 ist eine Ausführungsform des Zwischenwirbelimplantates 1 zwischen zwei aneinander angrenzenden Wirbelkörpern implantiert. Die Orientierung der drei plattenförmigen Teile 10;20;30 ist derart, dass die Längsachse 41 des ersten kreiszylindrischen Stabes 40 anterior-posterior verläuft und die Längsachse 51 des zweiten kreiszylindrischen Stabes 50 lateral-lateral verläuft.

## Patentansprüche

1. Zwischenwirbelimplantat (1), insbesondere künstliche Bandscheibe, mit einer Zentralachse (2) sowie
A) einem oberen, plattenförmigen Teil (10), der für die Anlage an die Grundplatte eines darüber liegenden Wirbelkörpers geeignet ist, wobei das obere Teil (10) eine ventrale Seitenfläche (11), eine dorsale Seitenfläche (12), zwei laterale Seitenflächen (13;14), eine obere Appositionsfläche (15) und eine untere Oberfläche (16) aufweist;
B) einem unteren, plattenförmigen Teil (30), der für die Anlage an die Deckplatte eines darunter liegenden Wirbelkörpers geeignet ist, wobei das untere Teil (20) eine ventrale Seitenfläche (31), eine dorsale Seitenfläche (32), zwei laterale Seitenflächen (33;34), eine untere Appositionsfläche (35) und eine obere Oberfläche (36) aufweist,
wobei
C) zwischen dem oberen und unteren Teil (10;30) ein mittlerer, plattenförmiger Teil (20) angeordnet ist, wobei der mittlere Teil (20) eine ventrale Seitenfläche (21), eine dorsale Seitenfläche (22), zwei laterale Seitenflächen (23;24), eine untere, gegen das untere Teil (30) gerichtete Oberfläche (25) und eine obere, gegen das obere Teil (10) gerichtete Oberfläche (26) aufweist;
**dadurch** gekennzeichet, dass
D) zwischen dem oberen Teil (10) und dem mittleren Teil (20) ein erster kreiszylindrischer Stab (40) mit einer Längsachse (41) angeordnet ist; und
E) zwischen dem unteren Teil (30) und dem mittleren Teil (20) ein zweiter kreiszylindrischer Stab (50) mit einer Längsachse (51) angeordnet ist.

2. Zwischenwirbelimplantat (1) nach Anspruch 1, **dadurch gekennzeichnet, dass** die untere Oberfläche (16) des ersten Teils (10) und die obere Oberfläche (26) des mittleren Teils (20) als Gleitflächen für den sie berührenden ersten, kreiszylindrischen Stab (40) ausgebildet sind.

3. Zwischenwirbelimplantat (1) nach Anspruch 2, **dadurch gekennzeichnet, dass** die zwei Gleitflächen (16;26) für den ersten kreiszylindrischen Stab (40) als planare, kreiszylindrische oder kegelförmige Ebenen ausgebildet sind.

4. Zwischenwirbelimplantat (1) nach einem der Ansprüche 1 bis 3, **dadurch gekennzeichnet**, das die untere Oberfläche (25) des mittleren plattenförmigen Teils (20) und die obere Oberfläche (36) des unteren plattenförmigen Teiles (30) als Gleitflächen für den sie berührenden zweiten, kreiszylindrischen Stab (50) ausgebildet sind.

5. Zwischenwirbelimplantat (1) nach Anspruch 4, **dadurch gekennzeichnet, dass** die zwei Gleitflächen (25;36) für den zweiten, kreiszylindrischen Stab (50) als planare, kreiszylindrische oder kegelförmige Ebenen ausgebildet sind.

6. Zwischenwirbelimplantat (1) nach einem der Ansprüche 2 bis 5, **dadurch gekennzeichnet, dass** eine oder mehrere der Gleitflächen (16;26;25;36) mindestens partiell mit einer peripheren Umrandung (70) versehen sind.

7. Zwischenwirbelimplantat (1) nach einem der Ansprüche 2 bis 6, **dadurch gekennzeichnet, dass** auf einer oder mehreren der Gleitflächen (16;26;25;36) eine Anzahl Begrenzer/Stops (80) für die Rotation der zylindrischen Stäbe (40;50) um die Zentralachse (2) vorgesehen sind.

8. Zwischenwirbelimplantat (1) nach einem der Ansprüche 2 bis 6, **dadurch gekennzeichnet, dass** auf einem oder beiden der durch die vier Gleitflächen (16;26;25;36) gebildeten Gleitflächen-Paare (16;26; 25;36) ein Paar Nuten (17;27;28;37) als Lager für den ersten und/oder zweiten Stab (40;50) vorgesehen sind.

9. Zwischenwirbelimplantat (1) nach Anspruch 8, **dadurch gekennzeichnet, dass** das Paar Nuten (17;27;28;37) kongruent zu den darin aufzunehmenden, kreis-zylindrischen Stäben (40,50) ist.

10. Zwischenwirbelimplantat (1) nach Anspruch 8 oder 9, **dadurch gekennzeichnet, dass** mindestens ein Paar Nuten (17;27;28;37) inkongruent zu den darin aufzunehmenden, kreiszylindrischen Stäben (40,50) ausgestaltet sind und vorzugsweise eine Breite aufweisen, welche eine begrenzte Rotation der Stäbe (40;50) um die Zentralachse (2) in den Nuten (17;27;28;37) zulässt.

11. Zwischenwirbelimplantat (1) nach einem der Ansprüche 8 bis 10, **dadurch gekennzeichnet, dass** mindesten ein Teil der Nuten (17;27;28;37) eine peripher angebrachte Begrenzung/Stop (75) gegen eine axiale Verschiebung des darin aufgenommenen Stabes (40;50) aufweist.

12. Zwischenwirbelimplantat (1) nach einem der Ansprüche 8 bis 11, **dadurch gekennzeichnet, dass** das eine Paar Nuten (17;27) für den ersten Stab (40) von den ventralen zu den dorsalen Seitenflächen (11;21;31;12;22;32) der dazugehörenden plattenförmigen Teile (10;20;30) verlaufen und das zweite Paar Nuten (28;37) für den zweiten Stab (50) zwischen den lateralen Seitenflächen (13;14;23;24;33;34) der dazugehörenden plattenförmigen Teile (10;20;30) verlaufen.

13. Zwischenwirbelimplantat (1) nach Anspruch 7, **dadurch gekennzeichnet, dass** die Begrenzer/Stops (80) so angeordnet sind, dass die Längsachse (41) des ersten Stabes (40) die ventralen und dorsalen Seitenflächen (11;21;31;12;22;32) der dazugehörenden plattenförmigen Teile (10;20;30) schneiden, und dass die Längsachse (51) des zweiten Stabes (50) die lateralen Seitenflächen (13;14;23;24;33;34) der dazugehörenden plattenförmigen Teile (10;20;30) schneiden.

14. Zwischenwirbelimplantat (1) nach einem der Ansprüche 1 bis 13, **dadurch gekennzeichnet, dass** elastisch deformierbare Mittel (60) vorgesehen sind, welche das obere und untere plattenförmige Teil (10;30) mit dem dazwischen liegenden mittleren plattenförmigen Teil (20) und den beiden Stäben (40;50) zusammenhalten.

15. Zwischenwirbelimplantat (1) nach Anspruch 14, **dadurch gekennzeichnet, dass** die elastisch deformierbaren Mittel (60) Federn (61) oder elastomere Verbindungselemente sind.

16. Zwischenwirbelimplantat (1) nach einem der Ansprüche 2 bis 15, **dadurch gekennzeichnet, dass** die vier Gleitflächen (16;25;26;36) und die beiden Stäbe (40,50) metallisch sind.

17. Zwischenwirbelimplantat (1) nach einem der Ansprüche 2 bis 15, **dadurch gekennzeichnet, dass** die vier Gleitflächen (16;25;26;36) metallisch und die beiden Stäbe (40,50) keramisch sind.

18. Zwischenwirbelimplantat (1) nach einem der Ansprüche 1 bis 17, **dadurch gekennzeichnet, dass** Mittel (90) vorgesehen sind, welche geeignet sind eine temporäre Blockierung der Beweglichkeit der drei plattenförmigen Teile (10;20;30) relativ zueinander herbeizuführen.

19. Zwischenwirbelimplantat (1) nach Anspruch 18, **dadurch gekennzeichnet, dass** die Mittel (90) an den beiden ventralen Seitenflächen (11;21;31) an den drei plattenförmigen Teilen (10;20;30) anbringbar sind.

20. Zwischenwirbelimplantat (1) nach Anspruch 18 oder 19, **dadurch gekennzeichnet, dass** die Mittel (90) einen Einsatz (91) mit einem unteren Ende (95) und einem oberen Ende (96) und an den beiden aussenstehenden plattenförmigen Teilen (10;30) je eine Vertiefung (92;93) in den Oberflächen (16;36) umfassen, welche an den ventralen Seitenflächen (11;31) der beiden aussenstehenden plattenförmigen Teile (10;30) offen sind, und dass der Einsatz (91) mit seinen Enden (95;96) in je eine der beiden Vertiefung (92;93) einfügbar ist.

21. Zwischenwirbelimplantat (1) nach Anspruch 20, **dadurch gekennzeichnet, dass** die Vertiefungen (42;43) Schwalbenschwanzführungen sind und die Enden (45;46) am Einsatz (41) komplementär zu diesen Schwalbenschwanzführungen ausgestaltet sind.

22. Zwischenwirbelimplantat (1) nach Anspruch 21, **dadurch gekennzeichnet, dass** sich die Schwalbenschwanzführungen von den ventralen Seitenflächen (11;31) der beiden aussenstehenden plattenförmigen Teilen (10;30) her gegen die dorsalen Seitenflächen (12;32) der beiden aussenstehenden plattenförmigen Teile (10;30) verjüngen.

## Claims

1. Intervertebral implant (1), specifically an artificial intervertebral disk, with a central axis (2) and
A) an upper plate-shaped section (10), suitable for laying onto the base plate of a vertebral body lying above, wherein the upper section (10) is provided with a ventral side area (11), a dorsal side area (12), two lateral side areas (13;14), an upper apposition surface (15) and a lower surface (16);
B) and a lower plate-shaped section (30) suitable for laying onto the cover plate of a vertebral body lying below, wherein the upper section (20) is provided with a ventral side area (31), a dorsal side area (32), two lateral side areas (33;34), an upper apposition surface (35) and a lower surface (36),
whereby
C) between the upper and lower section (10;30), a central, plate-shaped section (20) is arranged, wherein the central section (20) is provided with a ventral side surface (21), a dorsal side surface (22), two lateral side surfaces (23;24), a lower surface (25) facing the lower section (30) and an upper surface (26) facing the upper section (10);
**characterised in that**
D) between the upper section (10) and the central section (20), a first circular-cylindrical rod (40) with a longitudinal axis (41) is arranged; and
E) between the lower section (30) and the central section (20), a second circular-cylindrical rod (50) with a longitudinal axis (51) is arranged.

2. Intervertebral implant (1) according to Claim 1, **characterised in that** the lower surface (16) of the first section (10) and the upper surface (26) of the central section (20) are formed as sliding surfaces for the first, circular-cylindrical rod (40) contacting them.

3. Intervertebral implant (1) according to Claim 2, **characterised in that** the two sliding surfaces (16;26) for the first circular-cylindrical rod (40) are formed as flat, circular-cylindrical or conical planes.

4. Intervertebral implant (1) according to one of the Claims 1 to 3, **characterised in that** the lower surface (25) of the central plate-shaped section (20) and the upper surface (36) of the lower plate-shaped section (30) are formed as sliding surfaces for the second, circular-cylindrical rod (50) contacting them.

5. Intervertebral implant (1) according to Claim 4, **characterised in that** the two sliding surfaces (25;36) for the second, circular-cylindrical rod (50) are formed as flat, circular-cylindrical or conical planes.

6. Intervertebral implant (1) according to one of the Claims 2 to 5, **characterised in that** one or more of the sliding surfaces (16;26;25;36) is provided at least partially with a peripheral bordering (70).

7. Intervertebral implant (1) according to one of the Claims 2 to 6, **characterised in that** a number of limits/stops (80) for the rotation of the cylindrical rods (40;50) around the central axis (2) are provided on one or more of the sliding surfaces (16;26;25;36).

8. Intervertebral implant (1) according to one of the Claims 2 to 6, **characterised in that** a pair of grooves (17;27;28;37) is provided as a bearing for the first and/or second rod (40;50) on one or both of the sliding surface pairs (16;26; 25;36) formed by the four sliding surfaces (16;26;25;36).

9. Intervertebral implant (1) according to Claim 8, **characterised in that** the pair of grooves (17;27;28;37) is congruent to the circular-cylindrical rods (40,50) carried therein.

10. Intervertebral implant (1) according to Claim 8 or 9, **characterised in that** at least one pair of grooves (17;27;28;37) is designed incongruent to the circular-cylindrical rods (40,50) it has to bear and is preferably provided with a width that allows a limited rotation of the rods (40;50) around the central axis (2) in the grooves (17;27;28;37).

11. Intervertebral implant (1) according to one of the Claims 8 to 10, **characterised in that** at least a part of the grooves (17;27;28;37) is provided with a limit/stop (75) attached on the periphery to prevent axial shifting of the rod (40;50) carried therein.

12. Intervertebral implant (1) according to one of the Claims 8 to 11, **characterised in that** the one pair of grooves (17;27) for the first rod (40) extends from the ventral to the dorsal side surfaces (11;21;31;12;22;32) of the corresponding plate-shaped sections (10;20;30) and the second pair of grooves (28;37) for the second rod (50) extends between the lateral side surfaces (13;14;23;24;33;34) of the corresponding plate-shaped sections (10;20;30).

13. Intervertebral implant (1) according to Claim 7, **characterised in that** the limits/stops (80) are arranged so that the longitudinal axis (41) of the first rod (40) intersects the ventral and dorsal side surfaces (11;21;31;12;22;32) of the corresponding plate-shaped sections (10;20;30), and that the longitudinal axis (51) of the second rod (50) intersects the lateral side surfaces (13;14;23;24;33;34) of the corresponding plate-shaped sections (10;20;30).

14. Intervertebral implant (1) according to one of the Claims 1 to 13, **characterised in that** an elastically deformable means (60) is provided that holds the upper and lower plate-shaped sections (10;30) together with the intermediate lying central plate-shaped section (20) and the two rods (40;50).

15. Intervertebral implant (1) according to Claim 14, **characterised in that** the elastically deformable means (60) is provided as springs (61) or elastomer connection elements.

16. Intervertebral implant (1) according to one of the Claims 2 to 15, **characterised in that** the four sliding surfaces (16;25;26;36) and the two rods (40,50) are made of metal.

17. Intervertebral implant (1) according to one of the Claims 2 to 15, **characterised in that** the four sliding surfaces (16;25;26;36) are made of metal and the two rods (40,50) are made of ceramic.

18. Intervertebral implant (1) according to one of the Claims 1 to 17, **characterised in that** means (90) are provided that are suitable to create temporary blocking of the mobility of the three plate-shaped sections (10;20;30) relative to each other.

19. Intervertebral implant (1) according to Claim 18, **characterised in that** the means (90) can be attached to the three plate-shaped sections (10;20;30) on the two ventral side surfaces (11;21;31).

20. Intervertebral implant (1) according to Claim 18 or Claim 19, **characterised in that** the means (90) comprise an insert (91) with a lower end (95) and an upper end (96) and a depression (92;93) in the surfaces (16;36) on each of the two external plate-shaped sections (10;30), which are open on the ventral side surfaces (11;31) of the two external plate-shaped sections (10;30), and that the insert (91) can be inserted with its ends (95;96) into each of the two depressions (92;93).

21. Intervertebral implant (1) according to Claim 20, **characterised in that** the depressions (42;43) are dovetail guides and the ends (45;46) on the insert (41) are configured complementary to these dovetail guides.

22. Intervertebral implant (1) according to Claim 21, **characterised in that** the dovetail guides are tapered from the ventral side surfaces (11;31) of the two external plate-shaped section (10;30) towards the dorsal side surfaces (12;32) of the two external plate-shaped sections (10;30).

## Revendications

1. Implant intervertébral (1), en particulier disque intervertébral artificiel, présentant un axe central (2) ainsi que
A) une partie supérieure en forme de plaque (10) appropriée à l'appui sur le plateau inférieur d'un corps vertébral **sus-jacent,** la partie supérieure (10) présentant une face latérale ventrale (11), une face latérale dorsale (12), deux faces latérales sur les côtés (13 ; 14), une surface d'apposition supérieure (15) et une surface inférieure (16) ;
B) une partie inférieure en forme de plaque (30) appropriée à l'appui sur le plateau supérieur d'un corps vertébral sous-jacent, la partie inférieure (30) présentant une face latérale ventrale (31), une face latérale dorsale (32), deux faces latérales sur les côtés (33 ; 34), une surface d'apposition inférieure (35) et une surface supérieure (36);
dans lequel
C) une partie intermédiaire en forme de plaque (20) est disposée entre la partie supérieure et la partie inférieure (10 ; 30), la partie intermédiaire (20) présentant une face latérale ventrale (21), une face latérale dorsale (22), deux faces latérales sur les côtés (23 ; 24), une surface inférieure (25) dirigée vers la partie inférieure (30) et une surface supérieure (26) dirigée vers la partie supérieure (10),
**caractérisé en ce que**
D) une première barre en forme de cylindre circulaire (40) ayant un axe longitudinal (41) est disposée entre la partie supérieure (10) et la partie intermédiaire (20) ; et
E) une deuxième barre en forme de cylindre circulaire (50) ayant un axe longitudinal (51) est disposée entre la partie inférieure (30) et la partie intermédiaire (20).

2. Implant intervertébral (1) selon la revendication 1, **caractérisé en ce que** la surface inférieure (16) de la première partie (10) et la surface supérieure (26) de la partie intermédiaire (20) sont réalisées en tant que surfaces de glissement pour la première barre en forme de cylindre circulaire (40) en contact avec celles-ci.

3. Implant intervertébral (1) selon la revendication 2, **caractérisé en ce que** les deux surfaces de glissement (16 ; 26) pour la première barre en forme de cylindre circulaire (40) sont réalisées en tant que plans planaires, en forme de cylindre circulaire ou sous forme conique.

4. Implant intervertébral (1) selon l'une quelconque des revendications 1 à 3, **caractérisé en ce que** la surface inférieure (25) de la partie intermédiaire en forme de plaque (20) et la surface supérieure (36) de la partie inférieure en forme de plaque (30) sont réalisées en tant que surfaces de glissement pour la deuxième barre en forme de cylindre circulaire (50) en contact avec celles-ci.

5. Implant intervertébral (1) selon la revendication 4, **caractérisé en ce que** les deux surfaces de glissement (25 ; 36) pour la deuxième barre en forme de cylindre circulaire (50) sont réalisées en tant que plans planaires, en forme de cylindre circulaire ou sous forme conique.

6. Implant intervertébral (1) selon l'une quelconque des revendications 2 à 5, **caractérisé en ce qu'**une ou plusieurs des surfaces de glissement (16; 26 ; 25 ; 36) sont pourvues, au moins en partie, d'un bord périphérique (70).

7. Implant intervertébral (1) selon l'une quelconque des revendications 2 à 6, **caractérisé en ce qu'**un certain nombre de limiteurs/butées (80) pour la rotation des barres en forme de cylindre circulaire (40 ; 50) autour de l'axe central (2) sont prévus sur une ou plusieurs des surfaces de glissement (16 ; 26 ; 25 ; 36).

8. Implant intervertébral (1) selon l'une quelconque des revendications 2 à 6, **caractérisé en ce qu'**une paire de rainures (17 ; 27; 28 ; 37) est prévue en tant que palier pour la première et/ou la deuxième barre (40 ; 50) sur l'une ou les deux paires de surfaces de glissement (16 ; 26 ; 25 ; 36) formées par les quatre surfaces de glissement (16 ; 26 ; 25 ; 36).

9. Implant intervertébral (1) selon la revendication 8, **caractérisé en ce que** la paire de rainures (17 ; 27 ; 28 ; 37) est congruente avec les barres en forme de cylindre circulaire (40 ; 50) destinées à être logées dans celles-ci.

10. Implant intervertébral (1) selon la revendication 8 ou 9, **caractérisé en ce qu'**au moins une paire de rainures (17 ; 27 ; 28 ; 37) est conçue de manière non congruente avec les barres en forme de cylindre circulaire (40 ; 50) destinées à être logées dans celles-ci, et **en ce qu'**elle présente de préférence une largeur qui autorise une rotation limitée des barres (40 ; 50) dans les rainures (17 ; 27 ; 28 ; 37) autour de l'axe central (2).

11. Implant intervertébral (1) selon l'une quelconque des revendications 8 à 10, **caractérisé en ce qu'**au moins une partie des rainures (17 ; 27 ; 28 ; 37) présente un limiteur/butée périphérique (75) empêchant un déplacement axial de la barre (40 ; 50) logée dans celles-ci.

12. Implant intervertébral (1) selon l'une quelconque des revendications 8 à 11, **caractérisé en ce que** la première paire de rainures (17 ; 27) pour la première barre (40) s'étend des faces latérales ventrales jusqu'aux faces latérales dorsales (11 ; 21 ; 31 ; 12 ; 22 ; 32) des parties en forme de plaque correspondantes (10 ; 20 ; 30), et **en ce que** la deuxième paire de rainures (28 ; 37) pour la deuxième barre (50) s'étend entre les faces latérales sur les côtés (13 ; 14 ; 23 ; 24 ; 33 ; 34) des parties en forme de plaque correspondantes (10 ; 20 ; 30).

13. Implant intervertébral (1) selon la revendication 7, **caractérisé en ce que** les limiteurs/butées (80) sont placés de telle sorte que l'axe longitudinal (41) de la première barre (40) coupe les faces latérales ventrales et dorsales (11 ; 21 ; 31 ; 12 ; 22 ; 32) des parties en forme de plaque correspondantes (10 ; 20 ; 30), et **en ce que** l'axe longitudinal (51) de la deuxième barre (50) coupe les faces latérales sur les côtés (13 ; 14 ; 23 ; 24 ; 33 ; 34) des parties en forme de plaque correspondantes (10 ; 20 ; 30).

14. Implant intervertébral (1) selon l'une quelconque des revendications 1 à 13, **caractérisé en ce que** des moyens déformables de manière élastique (60) sont prévus, lesquels moyens fixent la partie supérieure en forme de plaque et la partie inférieure en forme de plaque (10 ; 30) à la partie intermédiaire en forme de plaque (20) située entre elles et aux deux barres (40 ; 50).

15. Implant intervertébral (1) selon la revendication 14, **caractérisé en ce que** les moyens déformables de manière élastique (60) sont des ressorts (61) ou des éléments de liaison élastomères.

16. Implant intervertébral (1) selon l'une quelconque des revendications 2 à 15, **caractérisé en ce que** les quatre surfaces de glissement (16 ; 25 ; 26 ; 36) et les deux barres (40 ; 50) sont faites de métal.

17. Implant intervertébral (1) selon l'une quelconque des revendications 2 à 15, **caractérisé en ce que** les quatre surfaces de glissement (16 ; 25 ; 26 ; 36) sont faites de métal et les deux barres (40 ; 50) sont faites de céramique.

18. Implant intervertébral (1) selon l'une quelconque des revendications 1 à 17, **caractérisé en ce que** des moyens (90) sont prévus, lesquels moyens sont appropriés pour provoquer un blocage temporaire de la mobilité des trois parties en forme de plaque (10 ; 20 ; 30) les unes par rapport aux autres.

19. Implant intervertébral (1) selon la revendication 18, **caractérisé en ce que** les moyens (90) peuvent être disposés sur les trois faces latérales ventrales (11 ; 21 ; 31) des trois parties en forme de plaque (10 ; 20 ; 30).

20. Implant intervertébral (1) selon la revendication 18 ou 19, **caractérisé en ce que** les moyens (90) comprennent une pièce rapportée (91) présentant une extrémité inférieure (95) et une extrémité supérieure (96), et un évidement (92 ; 93) dans les surfaces (16 ; 36) sur chacune des deux parties en forme de plaque externes (10 ; 30), lequel évidement est ouvert au niveau des faces latérales ventrales (11 ; 31) des deux parties en forme de plaques externes (10 ; 30), et **en ce que** la pièce rapportée (91) peut être insérée, avec ses extrémités (95 ; 96), dans chacun des deux évidements (92 ; 93).

21. Implant intervertébral (1) selon la revendication 20, **caractérisé en ce que** les évidements (92 ; 93) sont des guidages en queue d'aronde et les extrémités (95 ; 96) au niveau de la pièce rapportée (91) sont conçues de manière complémentaire à ces guidages en queue d'aronde.

22. Implant intervertébral (1) selon la revendication 21, **caractérisé en ce que** les guidages en queue d'aronde rétrécissent en partant des faces latérales ventrales (11 ; 31) des deux parties en forme de plaque externes (10 ; 30) jusqu'aux faces latérales dorsales (12;32) des deux parties en forme de plaque externes (10 ; 30).
